# EUROPEAN PATENT SPECIFICATION

(11) **EP 3 013 279 B1**
(45) Date of publication and mention of the grant of the patent: **11.03.2020**
(21) Application number: 14742076.4
(22) Date of filing: 26.06.2014
(51) Int. Cl.: A61M 27/00, A61F 2/04, A61L 31/14, A61L 31/16

(54) **STENTS**
STENTS
ENDOPROTHÈSES

(30) Priority: 27.06.2013 US 201361839999 P
(43) Date of publication of application: 04.05.2016
(73) Proprietor: Boston Scientific Scimed, Inc., Maple Grove, MN 55311 (US)
(72) Inventor: HARRAH, Timothy, P., Cambridge, MA 02139 (US); LI, Jianmin, Lexington, MA 02421 (US); BODEN, Mark W., Harrisville, RI 02830 (US)
(74) Representative: Pfenning, Meinig & Partner mbB
(86) International application number: PCT/US2014/044349
(87) International publication number: WO 2014/210315

(56) References cited:
- EP-A1- 1 550 477
- US-A1- 2006 009 839

## Description

### TECHNICAL FIELD

The present disclosure generally relates to medical devices, and more particularly to ureteral stents for enhancing patient comfort.

### BACKGROUND

Ureteral stents may be used to create a pathway for urinary drainage from the kidney to the bladder in patients with ureteral obstruction or injury. Ureteral stents may also be used to enhance or otherwise protect the integrity of the ureter in a variety of surgical applications. A number of clinical conditions can produce interruption in urine flow including, for example, intrinsic obstruction of the ureter due to tumor growth, stricture or stones, compression of the ureter due to extrinsic tumor growth, stone fragment impaction in the ureter following extracorporeal shock wave lithotripsy, and ureteral procedures such as endopyelotomy and ureteroscopy. Stents may be used to treat or avoid obstructions of the ureter (such as ureteral stones or ureteral tumors) that disrupt the flow of urine from the corresponding kidney to the bladder. Serious obstructions of the urinary tract may cause urine to back up into the kidney and thereby threaten renal function. Ureteral stents may also be used after endoscopic inspection of the ureter.

Ureteral stents may be generally tubular in shape, terminating in two opposing ends: a kidney distal end and a urinary bladder proximal end. One or both of the ends of the stent may be coiled in a pigtail spiral or J-shape. This structure may reduce or prevent unintentional upward and/or downward migration of the stent in the lumen of the ureter, which may be caused by a patient's day-to-day physical activities. A kidney end coil is configured to retain the stent within the renal pelvis and to reduce or prevent unintended migration of the stent down the ureter. The bladder end coil is positioned in the bladder, and is configured to reduce or prevent unintended stent migration upward toward the kidney. The bladder end-coil also may be used to aid in retrieval and/or removal of the stent.

A ureteral stent may assist in the flow of urine from the kidney to the bladder. The region referred to as the ureteral vesical junction is a small area of the ureter that is immediately upstream, relative to normal urine flow, of the bladder. The ureteral vesicle junction is more sensitive, i.e., causes a greater pain sensation, relative to other regions of the ureter wall and kidneys, and therefore may be a source, in some cases a significant source, of patient discomfort when this region of the ureter contacts indwelling ureteral stents.

Ureteral stents, particularly the portion positioned in the ureter and proximal to the bladder, may produce adverse effects including hemorrhage, a continual urge to urinate, flank pain accompanying reflux of urine back up the ureter due to retrograde pressure when voiding, and/or trigone irritation resulting from chronic irritation due to the bladder-anchoring features of the stent or resulting from intraoperative trauma inflicted from passage of the device in the ureter. Ureteral stents may also cause encrustations, e.g., precipitation of salts from the urine along the device that may irritate tissue, foster bacterial growth, and/or hinder stent removal. In summary, stents may cause or contribute to patient discomfort and/or significant medical problems.

US 2006/0009839 relates to a composite vascular graft incorporating bioactive agents to deliver therapeutic materials and/or inhibit or reduce bacterial growth during and following the introduction of the graft to the implantation site in a vascular system. One or more biodegradable, bioactive agent coating layers are disposed over a graft member.

WO0050103 discloses a stent having a heterogeneous polymer composition and a variable degradation rate along its length such that retains its shape and stiffness during insertion into the body and can swell and soften inside the body to enhance patient comfort.

### BRIEF SUMMARY

Embodiments of the present disclosure relate to a medical device that may be used within a body tract such as a duct or lumen.

The invention is defined by the appended claims. Any subject matter referred as embodiment(s) and/or invention(s) which is/are not claimed do not form part of the invention. In at least one embodiment, the medical device comprises: an elongate member configured to be implanted within a patient's body, the elongate member including an outermost diameter and a plurality support members disposed within a biodegradable material; wherein degradation of the biodegradable material results in a reduction of the outermost diameter; and wherein at least two of the support members are arranged non-concentrically.

Embodiments of the medical device may include one or more of the following features: the elongate member may define a lumen therethrough; the lumen may be disposed centrally or offset of a longitudinal axis of the elongate member; the elongate member may not include a lumen therethrough; the at least two non-concentric support members may be arranged opposite each other across a central axis; the plurality of support members may include at least four support members disposed around a central axis; the plurality of support members may include at least six support members disposed around the central axis; the plurality of support members together may form a cylindrical shape; the plurality of support members may be connected at a proximal end of the support members, a distal end of the support members, or both the proximal end and the distal end of the support members; a first end of the elongate member may include a first anchoring mechanism and a second end of the elongate member may include a second anchoring mechanism, wherein at least one of the first and second anchoring mechanisms includes the biodegradable material; the biodegradable material may form the outermost diameter of the elongate member; a cross-section of the elongate member may have a non-circular shape; a cross-section of at least one of the support members may have a non-circular shape such as oval, triangular, square, rectangular, or star-shaped; the elongate member may include a therapeutic agent; the biodegradable material may include the therapeutic agent; or the medical device may have sufficient column strength for removal from the patient's body after degradation of the biodegradable material.

Embodiments of the present disclosure also include a medical device, comprising: an elongate member including at least four support members disposed around a central axis and a biodegradable material surrounding at least a portion of each of the support members; wherein at least two of the support members are arranged non-concentrically; and wherein the medical device is configured to be implanted within a ureter. Embodiments of the medical device may include one or more of the following features: the biodegradable material may completely surround an outer diameter of each of the support members; or the elongate member may include at least eight support members.

Embodiments of the present disclosure also include a medical device, comprising: an elongate member configured to be implanted within a patient's body, the elongate member including a plurality support members disposed within a biodegradable material and at least one therapeutic agent; wherein at least two of the support members are arranged non-concentrically. Embodiments of the medical device may include one or more of the following features: the biodegradable material may include the at least one therapeutic agent; the plurality of support members may include the at least one therapeutic agent; or the medical device may be configured to release at least 50% of the therapeutic agent within about 12 hours after implantation in the patient's body.

### BRIEF DESCRIPTION OF THE DRAWINGS

Embodiments of the present invention are described with reference to the following drawings for exemplary purposes only. In the drawings, like reference numerals refer to like parts throughout the various figures unless otherwise specified.

For a better understanding of the presently disclosed subject matter, reference will be made to the following Detailed Description, which is to be read in association with the accompanying drawings, wherein:
FIG. 1 shows a schematic view of an exemplary medical device, according to an embodiment of the present disclosure.
FIGS. 2A-2E show cross-sectional views of medical devices, in accordance with various embodiments of the present disclosure.
FIGS. 3A-3C show cross-sectional views of an exemplary medical device and gradual degradation of a portion of the device, according to an embodiment of the present disclosure.

### DETAILED DESCRIPTION

Embodiments of the present disclosure include medical devices, such as implantable medical devices, and methods of use thereof.

In some embodiments, the medical device comprises a ureteral stent, e.g., an implantable ureteral stent. The medical device may be configured to be implanted within a patient's body, and may include an elongate member configured to be implanted within a patient's body.

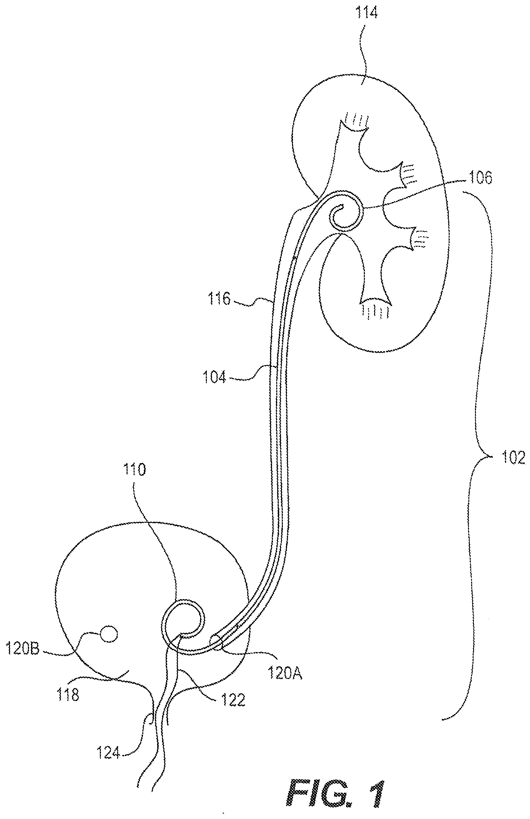

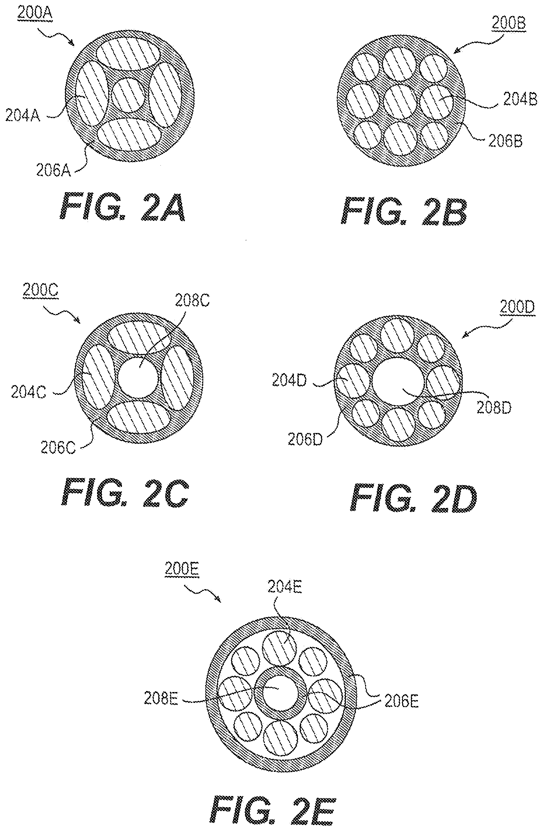

FIG. 1 is a schematic view of an exemplary ureteral stent 102 implanted within a patient's body according to an embodiment of the present disclosure. The ureteral stent 102 includes an elongate member 104 extending from a first end 106 (e.g., proximal end) to a second end 110 (e.g., distal end). While the first end 106, elongate member 104, and second end 110 of the stent 102 are shown as different portions (e.g., separated by lines in FIG. 1), it is understood that this is representative only such that different portions of the stent 102 may vary in length, size, and/or configuration. The ureteral stent 102 may include one or more anchoring mechanisms, for example at one or both of the first or second ends 106, 110. For example, as shown in FIG. 1, ureteral stent 102 may have an anchoring mechanism such as a pigtail or coil at each of the first end 106 and second end 110, e.g., a double pigtail configuration. The elongate member 104 may define an outermost diameter and include a plurality of support members or strands disposed within a matrix as discussed below with reference to FIGS. 2A-2E. In some embodiments, different portions of the stent 102 may include different materials. For example, the elongate member 104 may include one or more materials different from the first end 106 of the stent 102 and/or the second end 110 of the stent 102.

The elongate member 104 may define a lumen therethrough, e.g., along a longitudinal axis, such as a central axis of the elongate member 104. In some embodiments, the outermost diameter of the elongate member 104 may range from about 4-8 Fr, such as about 6 Fr, with a lumen diameter ranging from about 2-4 Fr, such as about 3 Fr. However, the elongate member 104 may have any other dimensions, regular or irregular, that are beneficial or otherwise useful according to the needs and preferences of a physician and/or patient.

The ureteral stent 102 as shown in FIG. 1 may be implanted within the body by any conventional method. For example, the ureteral stent 102 may be delivered into the body through the urethra 124 into bladder 118, and further pass through one of the ureteral orifices 120A or 120B within the bladder 118. For example, the first end 106 of the elongate member 104 may be passed partially or completely within ureter 116 through ureteral orifice 120A to reach the corresponding kidney 114.

After implantation, the first end 106 of the ureteral stent 102 may rest in a portion of the kidney 114, e.g., the renal kidney, as shown in FIG. 1. The second end 110 of the ureteral stent 102 may rest in the bladder 118. In some embodiments, however, the second end 110 of the ureteral stent 102 may rest proximate to the bladder 118, e.g., within the ureter 116. In some embodiments, for example, the first end 106 may include an anchoring mechanism to anchor the ureteral stent 102 in the kidney 114, and the second end 110 may rest within the ureter 116 without an anchoring mechanism.

In some embodiments, the ureteral stent 102 may be configured to facilitate removal from the body. For example, the ureteral stent 102 may include at least one thread 122 at the second end 110 (e.g., bladder end) to assist in removal of the stent 102 by pulling the thread 122. For example, single or multiple threads 122 may be attached to the ureteral stent 102 at the second end 110. While FIG. 1 shows a thread 122, embodiments of the present disclosure may include any other suitable structure or configuration to assist in stent removal. In at least some embodiments, at least a portion of the stent 102, e.g., the first end 106, second end 110, and/or elongate member 104, may be flexible.

FIGS. 2A-2E show cross-sectional views of exemplary elongate members 200A-200E, respectively, according to embodiments of the present disclosure. While elongate members 200A-200E have generally circular cross-sections or uniform outer diameters, the elongate member may have any suitable cross-section, including but not limited to, oval, triangular, square, rectangular, polygonal, etc. Thus, the cross-section of the elongate member may have a non-circular shape with a varying diameter. Further, the cross-section may be vary along the length of the elongate member, or may also be uniform along at least a portion or the entire length of the elongate member.

Referring generally to FIGS. 2A-2E, each elongate member 200 may include a plurality of support members 204 or strands disposed within a matrix 206 and extending along at least a portion of the length of the elongate member 200. In some embodiments, one or more of the support members 204 may extend the entire length of the elongate member 200 or the entire length of the stent 102. At least some of the elongate members 200 may include a lumen 208, e.g., an open lumen extending the entire length of the stent 102. Some or all of the support members 204, such as at least two of the support members 204, may be arranged or otherwise disposed non-concentrically. Further the support members may be of the same size and dimension as one another, or may have different sizes and/or dimensions.

One or more characteristics of the support members 204 (e.g., number, material(s), length, cross-sectional shape, volume, or other dimensions) may be chosen to correlate with, complement, conform to, or contrast with one or more characteristics of the matrix 206 (e.g., number of matrix regions, material(s), length, cross-sectional shape, volume, or other dimensions). Further, in some embodiments, characteristic(s) of the support members 204 and of the matrix 206 may be independently chosen. Thus, the support members 204 may, but need not be entirely disposed within the matrix 206, and the matrix 206 may, but need not surround one or more support members 204 at any given region or cross-sectional area of the stent 102. Thus, the area represented by matrix 206 in FIGS. 2A-2E may be filled entirely with one or more materials, or may be only partially filled with one or more materials. In some embodiments, for example, one or more matrix materials 206 may extend along at least a portion of the length of the stent 102. In other embodiments, one or more matrix materials 206 may extend along the entire length of the stent 102.

For example, FIG. 2A shows elongate member 200A with five support members 204A disposed in matrix 206A, wherein four of the support members having generally oval-shaped cross-sections are arranged in a circle (forming a generally cylindrical shape along a length of the elongate member 200A), and a fifth support member having a generally circular cross-section is disposed in the center of the elongate member 200A (e.g., along a longitudinal axis of elongate member 200A). At least one support member may be larger or smaller, e.g., may have a larger or smaller cross-section, than one or more other support members. For example, in FIG. 2A, the four oval-shaped support members 204A are larger than the fifth circular support member 204A.

In another embodiment shown in FIG. 2B, elongate member 200B includes nine support members 204B arranged 3x3 within matrix 206B. While each support member 204B as shown in FIG. 2B is generally of the same size as the other support members 204B, it is understood that each support member may have a different cross-sectional shape and/or size than one or more other support members. For example, in other embodiments, the size of the support members 204B may alternate from support members having a larger cross-section to those having a smaller cross-section. Further, any of the nine support members 204B may have a cross-sectional shape other than circular, e.g., oval, square, rectangular, triangular, star-shaped, or other regular or irregular shape.

While FIGS. 2A and 2B illustrate embodiments of elongate members 200A and 200B without open lumens, e.g., for the passage of a fluid therethrough, a portion of the elongate members 200A, 200B may dissolve over time to leave one or more open lumens and/or to reduce an external diameter of the elongate members 200A, 200B, thus allowing for the passage of fluid within and/or along the elongate members 200A, 200B.

FIG. 2C is a cross-sectional view of an exemplary configuration of an elongate member 200C that includes a plurality of generally oval-shaped support members 204C disposed in a matrix 206C and a lumen 208C. While the support member 240C are of generally the same cross-sectional size and shape, one or more of the support members 204C may be larger or smaller than one or more other support members 204C, and may have a different cross-sectional shape. The matrix 206C may include one or more biodegradable or resorbable materials. As shown in this embodiment, the lumen 208C may be open and thus not filled with the biodegradable material, thereby allowing for the passage of urine and/or other waste products therethrough. While FIG. 2C illustrates an open lumen 208C located at the center of elongate member 200C, in other embodiments the lumen 208C may be off-center of the longitudinal axis of the elongate member 200C. While lumen 208C has a generally circular cross-section as shown in FIG. 2C, other sizes or dimensions, such as a non-circular cross-section, are possible. The lumen 208C may be of comparable size to one or more of the support members 204C, or may be larger or smaller than one or more support members 204C.

FIG. 2D is a cross-sectional view of an exemplary configuration of an elongate member 200D that includes a plurality of generally circular support members 204D disposed in a matrix 206D. The support members 204D need not be circular, however; one or more support members 204D may have a non-circular cross-sectional shape. The support members 204D may have different sizes or dimensions from one other. For example, one or more support members 204D may have a larger cross-sectional size or area than one or more other support members 204D. The elongate member 200D also includes an open lumen 208D. The lumen 208D may be empty to allow passage of urine or other waste products therethrough. The lumen 208D may be of comparable size to one or more of the support members 204D, or may be larger or smaller. The lumen 208D need not be circular, but may have a regular or irregular cross-sectional shape that is non-circular.

In some embodiments, the matrix 206 may be arranged in portions or layers around some or all of the plurality of support members 204, e.g., forming a non-continuous layer. For example, FIG. 2E is a cross-sectional view of an exemplary configuration of an elongate member 200E including a plurality of support members 204E and a matrix 206E. The support members 204E may have the same or different sizes or dimensions from one other. For example, each of the support members 204E may have a generally circular cross-section as shown in FIG. 2E, or one or more support members 204E may have a non-circular cross-sectional shape. Further, the support members 204E may be of generally the same size, or one or more support members 204E may have a larger cross-sectional size or area than one or more other support members 204E.

As shown in FIG. 2E, biodegradable or resorbable material of the matrix 206E may form concentric tubes or lumens and thus not extend throughout the entire interior of the elongate member 200E around the support members 204E, thereby limiting the amount of matrix material 206E included in the elongate member 200E of the medical device. In other embodiments, the elongate member 200E may include discrete portions of matrix material 206E rather than continuous layers. The biodegradable material may be applied to the elongate member 200E by any suitable or beneficial method, such as by dip coating, to form a comparatively thin conformal coating. A lumen 208E (which may, but need not be, provided at a central axis) may be coated or may remain partially coated with the biodegradable material.

The examples shown in FIGS. 2A-2E illustrate different embodiments with the understanding that additional embodiments may be contemplated with any number of support members 204. For example, in some embodiments, the plurality of support members 204 may include 2, 3, 4, 5, 6, 7, 8, 9, 10, 11, or even 12 or more support members 204. Support members 204 and matrices 206 according to the present disclosure may be of any suitable size or dimension, and may take any combination of dimensions, e.g., one or more small support members 204 in combination with one or more large support members 204, a greater amount of support member material than matrix material or greater amount of matrix material than support member material for a given region or cross-sectional area of the stent 102. Any of the features discussed herein in connection to an embodiment may be used in combination with one or more features of any other embodiment. Further, other embodiments of the present disclosure will be apparent to those skilled in the art from consideration of the specification and practice of the embodiments disclosed herein.

The support members 204 and matrix 206 may comprise different materials. For example, in some embodiments, at least one support member 204 may include a non-biodegradable material and the matrix 206 may include a biodegradable material. In other embodiments, at least one support member 204 may include a biodegradable material and the matrix 206 may include a non-biodegradable material. Further, one or more support members 204 of the plurality of support members may comprise a material different from the other support members.

In some embodiments, the matrix 206 may include a biodegradable material. The biodegradable material may be resorbable and dissolve over time. Thus, in some embodiments, the medical device may have sufficient column strength for implantation, e.g., before dissolution of any biodegradable material of the matrix 206, and may become smaller, softer, and/or significantly more flexible and thus more comfortable over time. In some embodiments, dissolution of the biodegradable material of the matrix 206 provides an erodible surface that may resist protein deposition, bacterial adhesion, and/or mineralization.

As discussed above, each of the elongate members 200A-200E in FIGS. 2A-2E may include one or more non-degradable or permanent support members 204 disposed within a resorbable or degradable matrix 206. Examples of materials suitable for the support members 204 and/or matrix 206 according to the present disclosure include, but are not limited to, polyvinyl alcohol (PVA) and copolymers thereof, alginate, polylactic acid (PLA) and copolymers thereof, polydioxanone (PDO) and copolymers thereof, poly(caprolactone) and copolymers thereof, ethylene vinyl acetate (EVA), polyurethane (PU), silicone, polyisobutylene polyurethane (PIB-PUR), styrene isoprene butadiene and other styrene block copolymer elastomers, styrene-b-isobutylene-b-styrene (SIBS), acrylic polymers and copolymers, glycol polymers and copolymers such as, e.g., pluronics, poly(carbonates), poly(amides), poly(ureas), poly(anhydrides), amino acid-based polymers such as, e.g., tyrosine poly(carbonate) and tyrosine esters, and thermoplastic materials.

Further, the support members 204 and/or matrix 206 may include a material responsive to one or more stimuli such as pH, temperature (e.g., heat or cold), humidity (e.g., presence or absence of water or other solution), light, pressure, or composition. The stimuli may be, e.g., physical or chemical, and may act as a triggering agent to initiate or terminate dissolution or degradation of the material. In some embodiments, materials may interact with each other and/or with the body or a body material. For example, a material of a support member or matrix may interact with another material within the same or a different support member or matrix. Interactions between materials may result in physical and/or chemical change(s), and may result in a new mixture or composition. In some embodiments, a material or combination of materials within the stent 102 (e.g., a support member 204 and/or matrix 206 material) may be configured to interact with the body or a body material, such as to change or otherwise affect the composition, color, or fluidity of urine. Further, materials of the stent 102 may be configured to interact with each other and/or with the body or a body material at certain times after implantation of the stent 102 within the body, e.g., during degradation of a matrix 206 material and/or support member 204 material.

For example, in some embodiments, the matrix 206 may include one or more of PVA, alginate, PLA, PDO, or other material(s), and the support members 204 may include one or more of EVA, PU, silicone, PIB-PUR, SIBS, or other material(s). In other embodiments, the matrix 206 may include one or more of EVA, PU, silicone, PIB-PUR, SIBS, or other material(s), and the support members 204 may include one or more of PVA, alginate, PLA, PDO, or other material(s). The embodiments illustrated in FIGS. 2A-2E are intended to include support members 204 of any shape, size, material, etc., that enhances comfort and/or reduces or prevents encrustation. The biodegradable or resorbable material of the matrix may dissolve over time to form one or more lumens 208 within elongate member 200.

In some embodiments, the matrix 206 and/or one or more support members 204 may include at least one active pharmaceutical ingredient (API) or therapeutic agent, such as, e.g., an antibiotic, analgesic, anesthetic, estrogen, muscle relaxant, etc. Any suitable API and/or therapeutic agent may be included in the medical devices disclosed herein. In some embodiments, the support members 204 themselves may at least partially be formed of resorbable or biodegradable material and may include at least one API or therapeutic agent. Release of the therapeutic agent may be affected by one or more stimuli such as pH, temperature (e.g., heat or cold), humidity (e.g., presence or absence of water or other solution), light, pressure, or composition of the stent 102 such as composition of the support members 204 and/or matrix 206. The stimuli affecting the release of the therapeutic agent may be, e.g., physical or chemical, and may initiate, accelerate, inhibit, or terminate release of the therapeutic agent.

In some embodiments of the present disclosure, the medical device, e.g., ureteral stent, may be configured to release an amount of the therapeutic agent within a certain period of time. In an embodiment, for example, the medical device may be configured to release at least 25% of the therapeutic agent within about 12 hours after implantation within the patient's body. For example, the API or therapeutic agent may be included in the biodegradable material, and thus gradually release into the body as the biodegradable material dissolves over time. In some embodiments, for example, the medical device may be configured to release at least 25%, at least 50%, at least 60%, at least 75%, or even at least 80% or more of the therapeutic agent within a certain period of time, e.g., within about 1 hour, about 4 hours, about 6 hours, about 8 hours, about 12 hours, about 24 hours, about 36 hours, or about 48 hours after implantation. In some embodiments, the medical device may be configured to release at least 50% of the therapeutic agent with a period of time ranging from about 1 hour to about 48 hours after implantation, e.g., from about 12 hours to about 24 hours after implantation.

Further, release of a therapeutic agent may correspond to the activity of the therapeutic agent and/or correlate with a certain point of treatment after implantation of the stent 102 in the body. For example, the stent 102 may be configured to release a first therapeutic agent at a certain time after implantation and release a second therapeutic agent at a different time. In some embodiments, the stent 102 may be configured to release an analgesic agent within a first period of time after implantation, and then to release an antibiotic agent within a second period of time after implantation, or vice-versa. Release of a therapeutic agent may correlate with a body function, e.g., release of an antibiotic may correlate with stone expulsion. The stent 102 may be configured to indicate the presence, absence, and/or amount of therapeutic agent released. For example, one or more materials of the stent 102 may interact with each other, with the body or a body material, or with the therapeutic agent as an indication of release of the therapeutic agent. In some embodiments, one or more materials of the stent 102 may cause the urine to change in color, composition, fluidity, or affect another characteristic of the urine to indicate initial release or depletion of the therapeutic agent.

The support members 204 of the elongate member 200 may be directly and/or indirectly coupled or connected to one another and may remain disposed in the matrix 206. For example, two or more support members 204 may be coupled at a proximal end and/or a distal end of each support member via any suitable method including, but not limited to, adhesive bonding, tying, etc.

In some embodiments, one or more support members may be integral with a first end 106 and/or second end 110 of the stent 102 with reference to FIG. 1. For example, the first end 106 of the stent 102 may include a non-biodegradable material section (e.g., formed into a coil or other anchoring mechanism) that transitions into a plurality of support members that are embedded within a biodegradable matrix material to form the elongate member 104 that then transitions into a non-biodegradable section at the second end 110. Upon partial or complete degradation of the matrix material, for example, the non-biodegradable material at the second end 110 may act as a tail, e.g., to assist in removal of the stent 102 from the body. The non-biodegradable tail at the second end 110 may include a flexible material. Reducing the cross-sectional area of the first end 106 and/or second end 110 of the stent 102 through degradation of material may reduce patient pain or discomfort. For example, the second (bladder) end 110 may decrease in cross-sectional area (e.g., outer diameter) over time to decrease patient discomfort, for example at the ureterovesicular junction, e.g., vesicoureteral reflux, and/or at the trigone of the bladder.

In some embodiments, the plurality of support members may be connected at a proximal end, e.g., towards the renal kidney 114, and may not be connected at a distal end, e.g., towards the bladder 118. For example, as biodegradable material surrounding the plurality of support members dissolves over time, the distal end of the support members may become free to move relative to one another, thus enhancing patient comfort.

As mentioned above and shown in FIG. 1, the stent 102 may include one or more anchoring mechanisms. For example, the first end 106 of stent 102 may include a first anchoring mechanism, and the second end 110 may include a second anchoring mechanism. In some embodiments, at least a portion of the first and/or the second anchoring mechanisms may include biodegradable material. The first and second anchoring mechanisms may be in the form of pigtails. This pigtail structure may reduce or prevent the stent 102 from becoming unintentionally displaced or removed.

Biodegradable material may form the outermost diameter of the elongate member 104 (or 200A-200E shown in Figs. 2A-2E). The elongate member 104 may include at least one API (such as a drug) or other therapeutic agent along with the biodegradable material. Embodiments may include any useful or beneficial API or therapeutic agent including, but not limited to anesthetics, analgesics, muscle relaxants, etc. The therapeutic agent or API may be released into the body from the matrix 206 and/or from one or more of the support members 204. For example, in some embodiments, the biodegradable material of the matrix 206 dissolves over time to deliver a first API, e.g., an antibiotic, to the body for a certain period of time, such as twelve hours, after implantation of the stent 102 One or more of the support members 204 may then release a second API, e.g., an analgesic, into the body.

In some embodiments, as mentioned above in connection to FIG. 1, the ureteral stent 102 may have a double pigtail configuration, e.g., two anchoring mechanisms. A biodegradable material such as, e.g., PVA, alginate, PLA, PDO, or similar material may form all or part of one or both of the anchoring mechanisms. For example, one or more of the pigtails (or first and second anchoring mechanisms at first and second ends 106 and 110, respectively) may be at least partially formed of a biodegradable material. The softening of a renal pigtail, i.e., at the first end 106, may promote or otherwise facilitate migration of the stent 102 in a distal direction, e.g., such as at a desired time. The softening of the bladder pigtail, i.e., at the second end 110, may further enhance patient comfort.

As shown in FIGS. 2A-2E, the material, size, and number of support members 204, as well as the type of material of the matrix 206 and its exterior thickness, can affect various aspects, including performance aspects, of the ureteral stent 102. For example, varying the composition and configuration of the elongate member 104 may affect the placement and final strength of the stent 102, and the amount of matrix 206 present and properties of the material of the matrix 206 may affect such parameters as the duration, rate, effectiveness, etc., of the patient's body to break down the material and/or the release of any API and/or therapeutic agent. In addition, both the matrix 206 and some percentage or portion of the support members 204 (or fibers) may be resorbable. This may provide further control of the mechanical properties of the implant of the stent 102 over time.

FIGS. 2A-2E generally depict the support members 204 as being generally spherical or oval in shape. However, the support members 204 may have any shape, such as any spinnable cross-section, and the matrix 206 may be added as a conformal coating or coextruded in a more controlled profile. In addition, the material of the matrix 206 may be applied to only certain portions of the support members, such as to only the exterior of the support members 204.

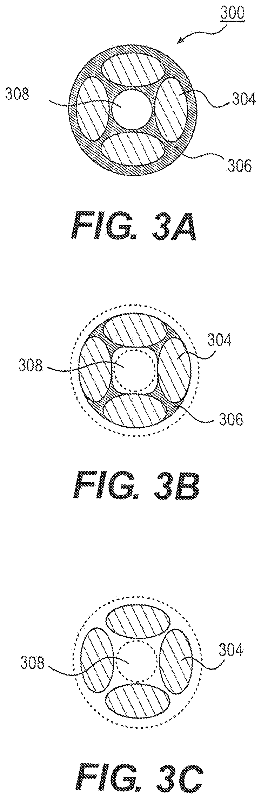

FIGS. 3A-3C depict various stages of degradation of biodegradable material of matrix 306 of stent 300 over time. FIG. 3A shows a cross-sectional view of elongate member 300 of a ureteral stent in accordance with an embodiment of the present disclosure. As shown, the elongate member 300 includes a matrix 306 of biodegradable material. The elongate member 300 includes an outermost diameter, and a plurality of support members 304 disposed within the biodegradable material of matrix 306. The support members 304 may have any suitable shape or size. The support members 304 also may be formed at least partially of a biodegradable material.

FIG. 3B is a cross-sectional view of elongate member 300 showing partial loss or degradation of biodegradable material of matrix 306 of FIG. 3A after being implanted in the body for a given period of time, e.g., 12 hours. The shape of the outermost diameter and central lumen 308 changes with degradation of the biodegradable material. For example, the outermost diameter of the elongate member 300 may decrease over time. Further, the size of the central lumen 308 may increase in over time. Degradation of the biodegradable material may soften the elongate member 300 and thereby enhance comfort for the patient.

FIG. 3C is a cross-sectional view showing a completely or substantially dissolved or degraded biodegradable material of matrix 306 of FIGS. 3A and 3B after being implanted for additional time, e.g., several days or weeks. The support members 304 are not degraded because they are formed (or substantially formed) of a non-biodegradable material. However, as indicated above, the support members 304 of some embodiments may at least partially be formed of biodegradable material. Degradation of biodegradable material of the elongate member 300 may result in a reduction of its outermost diameter.

While the embodiments described above are generally directed to a ureteral stent, the present disclosure is not so limited and may be applicable to other medical devices.

Moreover, while specific embodiments may have been illustrated and described collectively herein, it should be appreciated that any subsequent arrangement designed to achieve the same or similar purpose may be substituted for the specific embodiments described and shown herein. This disclosure is intended to cover any and all subsequent adaptations or variations of various embodiments. Combinations of the above embodiments, and other embodiments not specifically described herein, will be apparent to those of skill in the art upon reviewing the description.

Other embodiments of the present disclosure will be apparent to those skilled in the art from consideration of the specification and practice of the embodiments disclosed herein. It is intended that the specification and examples be considered as exemplary only, and departure in form and detail may be made without departing from the scope of the present disclosure as defined by the following claims.

## Claims

1. A medical device, comprising:
an elongate member (104) configured to be implanted within a patient's body, the elongate member (104) including an outermost diameter and a plurality of support members disposed within a matrix (206) of biodegradable or resorbable material;
wherein degradation of the biodegradable or resorbable material results in a reduction of the outermost diameter; and
wherein at least two of the support members (204) are arranged non-concentrically; and
wherein the elongate member (104) does not include a lumen.

2. The medical device of claim 1, wherein the at least two non-concentric support members (204) are arranged opposite each other across a central axis.

3. The medical device of claim 1, wherein the plurality of support members includes at least four support members disposed around a central axis.

4. The medical device of claim 1, wherein the plurality of support members together form a cylindrical shape.

5. The medical device of claim 1, wherein the plurality of support members (204) are connected at a proximal end of the support members (204), a distal end of the support members, or both the proximal end and the distal end of the support members (204).

6. The medical device of claim 1, wherein a first end (106) of the elongate member (104) includes a first anchoring mechanism and a second end (110) of the elongate member includes a second anchoring mechanism, wherein at least one of the first and second anchoring mechanisms includes the biodegradable or resorbable material.

7. The medical device of claim 1, wherein the biodegradable or resorbable material forms the outermost diameter of the elongate member (104).

8. The medical device of claim 1, wherein a cross-section of the elongate member (104) has a non-circular shape.

9. The medical device of claim 1, wherein the elongate member (104) includes a therapeutic agent.

10. The medical device of claim 9, wherein the biodegradable or resorbable material includes the therapeutic agent.

11. The medical device of claim 1, wherein at least one support member (204) has a non-circular shape.

12. The medical device of claim 1, wherein the plurality of support members (204) includes the at least one therapeutic agent.

13. The medical device including a therapeutic agent of claim 11, configured to release at least 50% of the therapeutic agent within about 12 hours after implantation in the patient's body.

## Patentansprüche

1. Medizinische Vorrichtung, umfassend:
ein längliches Element (104), das konfiguriert ist, um in den Körper eines Patienten implantiert zu werden, wobei das längliche Element (104) einen äußersten Durchmesser und eine Vielzahl von Stützelementen, die innerhalb einer Matrix (206) aus biologisch abbaubarem oder resorbierbarem Material eingerichtet sind, beinhaltet;
wobei der Abbau des biologisch abbaubaren oder resorbierbaren Materials in einer Reduktion des äußersten Durchmessers resultiert; und
wobei mindestens zwei der Stützelemente (204) nichtkonzentrisch angeordnet sind; und
wobei das längliche Element (104) kein Lumen beinhaltet.

2. Medizinische Vorrichtung nach Anspruch 1, wobei die mindestens zwei nichtkonzentrischen Stützelemente (204) einander gegenüberliegend über eine Mittelachse angeordnet sind.

3. Medizinische Vorrichtung nach Anspruch 1, wobei die Vielzahl von Stützelementen mindestens vier Stützelemente beinhaltet, die um eine Mittelachse herum eingerichtet sind.

4. Medizinische Vorrichtung nach Anspruch 1, wobei die Vielzahl von Stützelementen gemeinsam eine zylindrische Form bilden.

5. Medizinische Vorrichtung nach Anspruch 1, wobei die Vielzahl von Stützelementen (204) an einem proximalen Ende der Stützelemente (204), einem distalen Ende der Stützelemente oder sowohl an dem proximalen Ende als auch an dem distalen Ende der Stützelemente (204) verbunden sind.

6. Medizinische Vorrichtung nach Anspruch 1, wobei ein erstes Ende (106) des länglichen Elements (104) einen ersten Verankerungsmechanismus beinhaltet und ein zweites Ende (110) des länglichen Elements einen zweiten Verankerungsmechanismus beinhaltet, wobei mindestens einer der ersten und zweiten Verankerungsmechanismen das biologisch abbaubare oder resorbierbare Material beinhaltet.

7. Medizinische Vorrichtung nach Anspruch 1, wobei das biologisch abbaubare oder resorbierbare Material den äußersten Durchmesser des länglichen Elements (104) bildet.

8. Medizinische Vorrichtung nach Anspruch 1, wobei ein Querschnitt des länglichen Elements (104) eine nichtkreisförmige Form aufweist.

9. Medizinische Vorrichtung nach Anspruch 1, wobei das längliche Element (104) ein Therapeutikum beinhaltet.

10. Medizinische Vorrichtung nach Anspruch 9, wobei das biologisch abbaubare oder resorbierbare Material das Therapeutikum beinhaltet.

11. Medizinische Vorrichtung nach Anspruch 1, wobei mindestens ein Stützelement (204) eine nichtkreisförmige Form aufweist.

12. Medizinische Vorrichtung nach Anspruch 1, wobei die Vielzahl von Stützelementen (204) das mindestens eine Therapeutikum beinhaltet.

13. Medizinische Vorrichtung nach Anspruch 11, die konfiguriert ist, um mindestens 50 % des Therapeutikums innerhalb von etwa 12 Stunden nach der Implantation in den Körper des Patienten freizusetzen.

## Revendications

1. Dispositif médical, comprenant :
un élément allongé (104) qui est configuré de manière à ce qu'il soit implanté à l'intérieur du corps d'un patient, l'élément allongé (104) incluant un diamètre le plus externe et une pluralité d'éléments de support qui sont disposés à l'intérieur d'une matrice (206) en un matériau biodégradable ou résorbable ; dans lequel :
la dégradation du matériau biodégradable ou résorbable aboutit à une réduction du diamètre le plus externe ; et dans lequel :
au moins deux des éléments de support (204) sont agencés de façon non concentrique ; et dans lequel :
l'élément allongé (104) n'inclut pas de lumière.

2. Dispositif médical selon la revendication 1, dans lequel les au moins deux éléments de support non concentriques (204) sont agencés à l'opposé l'un de l'autre ou les uns des autres par rapport à un axe central.

3. Dispositif médical selon la revendication 1, dans lequel la pluralité d'éléments de support inclut au moins quatre éléments de support qui sont disposés autour d'un axe central.

4. Dispositif médical selon la revendication 1, dans lequel les éléments de support de la pluralité d'éléments de support forment ensemble une forme cylindrique.

5. Dispositif médical selon la revendication 1, dans lequel les éléments de support de la pluralité d'éléments de support (204) sont connectés au niveau d'une extrémité proximale des éléments de support (204), au niveau d'une extrémité distale des éléments de support ou à la fois au niveau de l'extrémité proximale et de l'extrémité distale des éléments de support (204).

6. Dispositif médical selon la revendication 1, dans lequel une première extrémité (106) de l'élément allongé (104) inclut un premier mécanisme d'ancrage et une seconde extrémité (110) de l'élément allongé inclut un second mécanisme d'ancrage, dans lequel au moins l'un des premier et second mécanismes d'ancrage inclut le matériau biodégradable ou résorbable.

7. Dispositif médical selon la revendication 1, dans lequel le matériau biodégradable ou résorbable forme le diamètre le plus externe de l'élément allongé (104).

8. Dispositif médical selon la revendication 1, dans lequel une section en coupe transversale de l'élément allongé (104) présente une forme non circulaire.

9. Dispositif médical selon la revendication 1, dans lequel l'élément allongé (104) inclut un agent thérapeutique.

10. Dispositif médical selon la revendication 9, dans lequel le matériau biodégradable ou résorbable inclut l'agent thérapeutique.

11. Dispositif médical selon la revendication 1, dans lequel au moins un élément de support (204) présente une forme non circulaire.

12. Dispositif médical selon la revendication 1, dans lequel la pluralité d'éléments de support (204) inclut l'au moins un agent thérapeutique.

13. Dispositif médical incluant un agent thérapeutique selon la revendication 11, configuré de manière à ce qu'il libère au moins 50 % de l'agent thérapeutique à l'intérieur d'environ 12 heures après l'implantation dans le corps du patient.
